# EUROPEAN PATENT APPLICATION

(11) **EP 1 829 537 A1**
(43) Date of publication of application: **05.09.2007**
(21) Application number: 05811785.4
(22) Date of filing: 02.12.2005
(51) Int. Cl.: A61K 31/122

(54) **COMPOSITION FOR BODY FAT REDUCTION**

(30) Priority: 03.12.2004 JP 2004382296
(71) Applicant: FUJI CHEMICAL INDUSTRY CO., LTD., Nakaniikawa-gun, Toyama 930-0397 (JP)
(72) Inventor: TAKAHASHI, Jiro c/o FUJI CHEM. INDUSTRY CO., LTD., Nakaniikawa-gun, Toyama 9300397 (JP); YAMASHITA, Eiji c/o FUJI CHEM. INDUSTRY CO., LTD., Nakaniikawa-gun, Toyama 9300397 (JP); FUKAMAUCHI, Makoto, Suzuka-shi, Mie 5138505 (JP); TANAKA, Isao, Suzuka-shi, Mie 5138505 (JP)
(74) Representative: Beckmann, Claus
(86) International application number: PCT/JP2005/022218
(87) International publication number: WO 2006/059730

(57) **Abstract**

[Object]

An increase in body fatis one of main causes of life-style -related diseases such as hypertension, insulin tolerance and hyperlipemia. A composition for body fat reduction is provided which inhibits body fat from decreasing or decreasing which and which is effective in the treatment and prevention of adult diseases a cause of which is body fat. Also, provided are a medicine and a food each containing the composition.

[Means to Solve Object]

The composition for body fat reduction comprises (A) astaxanthin and (B) at least one of active ingredient, an adsorbent and a solubilizer, a composition comprises (A) *Haetmatococcus* alga extract containing astaxanthin and (B) at least one of active ingredient, an adsorbent and a solubilizer, and a medicine and food, each containing the composition are provided.

## Description

### [Technical Field]

The present invention relates to a composition for body fat reduction which contains astaxanthin as an effective ingredient, and relates to a composition for body fat reduction which can be obtained by pulverizing *Haetmatococcus* alga and subjecting the pulverizied *Haetmatococcus* alga to extraction with an organic solvent. These compositions are administered as a medicine or taken as food and drink whereupon the present invention relates to a medicine and a food which are effective in reducing body fat and in preventing, improvingt and treating of corpulence or its related diseases.

### [Background Art]

In recent years, life style-related diseases increased by causes of intake of high caloric diet, the change in life style, lack of exercise, stress, etc. and became a serious social problem. Especially, corpulence or an excess of body weight which the rate of body fat becomes high is a great problem that it amounts to more than 20 percent of the total Japanese people. Corpulence is meant the state of body wherein intake energy becomes more excessive than consumption energy and as the result fatty tissue accumulates in greater amount than the normal case. In case of Japanese people, it indicates an adult man having more than 25 percent of body fat while it does an adult woman having more than 30 percent of body fat. When a person becomes corpulent, body fat accumulates in large amount whereby a variety of diseases such as hyperpiesia, cardiovascular, hyperlipemia, arteriosclerosis, diabetes, etc. are caused and complications such as blood vessel injury, eyesight injury, nerve injury, lowering of resistance, etc. occur.
Many studies have been made about means to solve corpulence which is the main cause of life style-related diseases, and various cures such as a diet cure, an exercise cure, medical cure, etc. have been developed and carried out.

Although a diet cure and an exercise cure have been generally conducted as cure of corpulence, continuous calorie intake-restricting cure (diet) and exercise cure requiring time and space to conduct an adequate exercise are hard to be practically carried out in the modern society due to the increase in the intake of diet energy and the change in labor environment.

As the medical cure of corpulence, there have been conducted a method of promoting consumption energy by accelerating decomposition of fat in fatty tissue and a method of inhibiting energy of intake by preventing lipid, saccharides and the like from being absorbed in digestive canal or limitation of intake. However, the medical cure has some problems that it cannot be easily carried out for the following reasons:
An adequate dosage is required; side effects occur that excessive intake prevents the intake of necessary nutrients and the balance of nutrients to be absorbed is lost and that a doctor's prescription is required.

There has been sought a composition for body fat reduction which is derived from natural material, which has no side effect and which can be easily taken as a food. Body fat reducing agent comprising Garcinia cambogia and citrus (Patent literature 1), a method of administering conjugated linolic acid (Patent literature 2) and a method of administering vitamin D₃ (Patent literature 3) are known.

Astaxanthin is a kind of the same carotenoid with β-carotene and a red pigment which is widely distributed in natural origin especially in ocean as in crustaceans such as shrimp, crab, etc.; fishes such as a salmon, a porgy, etc.; algae such as green alga of Haematococcus, etc.; yeasts such as *Phaffia* red yeast, etc. and which has a rich experience as food. It is known to have antioxidative action that is about 1000 times as strong as vitamin E and about 40 times as strong as β-carotene.

As other functional characteristics which astaxanthin has, its anti-inflammatory action, anti-arteriosclerotic action, action against diabetes, action of protecting retina from photic injury, diurnal rhythm adjusting action, immunopotentiation action, anti-stress action, sperm's quality improving action, action of preventing urinary bladder cancer from being induced, etc. have been reported. Also, as its action toward exercise, action of increasing duration of muscle function (Patent literature 4) and effect of inhibitng the rise in lactic acid value in blood during exercise (Non-patent literature 1) recovery effect of fatigue after exercise (Non-patent literature 2) were reported.

However, report has not yet been made that astaxanthin and an extract from *Haetmatococcus* alga which was obtained by pulverizing *Haetmatococcus* alga and subjecting the pulverized *Haetmatococcus* alga to solvent extraction exert effect to reduce body fat and to prevent body fat from increasing and consequently they have therapy and preventive effects toward corpulence.
[Patent literature 1]: JP2001^321126 A
[Patent literature 2]: JP10-508189 A
[Patent literature 3]: JP3-3210156 A
[Patent literature 4]: JP2001-514215 A
[Non-patent literature 1]: Clinical medicine; 18 (9), 85-1100, 2002
[Non-patent literature 2]: Science of fatigue and rest; 18 (1), 35 - 46,2003

### [Disclosure of the invention]

### [Subject Matter to be Solved by the Invention]

An object of the present invention is to provide a composition having effects to reduce body fat and to prevent body fat from increasing, and consequently is directed to treatment, improvement and prevention of various kinds of diseases caused by corpulence originating in excess of body fat, and to appearance and maintenance of good-looking form by slim effect.

### [Means for Solving the Subject Matter]

As a result of having ardently studied to solve the above object, the present inventors have found that astaxanthin and an extract of *Haetmatococcus* alga which was obtained by pulverizing *Haetmatococcus* alga and subjecting the pulverized *Haetmatococcus* alga to solvent extraction show effects to reduce body fat and to prevent body fat from increasing and have completed the present invention.

That is, the present invention is:
(1) a composition for body fat reduction which contains astaxanthin as an effective ingredient,
(2) a composition for body fat reduction which is obtained by pulverizing *Haetmatococcus* alga and subjecting pulverized *Haetmatococcus* alga to solvent extraction,
(3) a composition for body fat reduction which comprises incorporating one or more of an active ingredient, an adsorbent and a solubilizer into any one of (1) - (2) compositions,
(4) a body fat reducing agent containing any one of (1) - (3) compositions,
(5) a food and drink for body fat reduction which contains any one of (1) - (3) compositions,
(6) a process for reducing body fat which comprises administering or taking any one of (1)- (3) compositions to a human.

### [The Best Mode of Carrying Out the Invention]

The composition for body fat reduction which contains astaxanthin as an effective ingredient as well as the composition for body fat reduction which is obtained by pulverizing *Haetmatococcus* alga and subjecting the pulverized *Haetmatococcus* alga to solvent extraction is administered or taken as a medicine or food and drink whereby body fat can be reduced and its increase can be prevented without accompanying any side effect, and there can be treated, improved and prevented diseases the cause of which is an excess of body fat or corpulence.

The term "astaxanthin" is meant one derived from natural origin and one obtained by synthesis. As one derived from natural origin, there can be taken crusts, eggs and organs of crustaceans such as shrimp, krill, crab and the like; skins and eggs of various fishes and shellfishes; algae such as green alga of Haematococcus, etc.; yeasts such as *Phaffia* red yeast, etc.; oceanic bacteria; and seed plants such as *Adonis amurensis* and *Ranunculus acris*. An extract from natural origin and a chemically synthesized product are put on the marketplace and hence they are easily available.

Astaxanthin can be obtained by cultivation of e.g. *Phaffia* red yeast, Hematococcus alga, oceanic bacteria, etc. in an appropriate medium in accordance with the known method. Green alga of Hematococcus is preferred from the viewpoints of easiness of cultivation and extraction, astaxanthin contained at the highest concentration and high productivity.
Hematococcus alga is one of green algae belonging to Volvocida, Chlamydomonadaceae. Since it is usually green alga, it has a high chlorophyl content and green color, and swims in water with two flagellums. But, it forms dormant spores under the state of starvation such as lack of nutrient source, the change in temperature or otherwise, resulting in high astaxanthin content and red and spherical form. Although Hematococcus in either state may be used in the present invention, it is preferable to use Hematococcus forming dormant spores because of high astaxanthin content.
As green algae belonging to genus Hematococcus, e.g. *Haematococcus pluvialis, Haematococcus lacustris, Haematococcus capensis, Haematococcus droebakensis* and *Haematococcus zimbabwiensis* may be taken. Among them *Haematococcus pluvialis* is the most preferred.

As the cultivation method of Hematococcus algae, a method cultivating Hematococcus algae using hermetic type of cultivation apparatus is preferable because there is no possibility for different kind of microorganisms to be mixed therein and propagate and because the possibility of other contaminants to be mixed therein is very little. For example, a cultivation process using a hermetic type of dome-like, conical or cylindrical cultivation apparatus wherein culture incuvators are equipped with an optionally movable gas ejector (WO 99/50384). a cultivation process wherein a light source is placed in a cultivation tank and cultivation is conducted under radiation of light from the inner part of a cultivation tank, a cultivation process using hermetic type of cylindrical, plate-like or tubular cultivation tank, and a method wherein cultivation is conducted under radiation with light having peak wavelength of below about 540 nm (JP2004-147641 A) are suitable.

As a method of obtaining extract from Hematococcus alga containing astaxanthin involving in the present invention, there are taken (1) a method wherein after Hematococcus has been dried and pulverized, a superclinical extraction is conducted with carbon dioxide as solvent for extraction and carbon dioxide is removed to obtain an axtract (superclinical extraction method) and (2) a method wherein Hematococcus algais pulverized and subjected to solvent extraction, and the solvent is removed to obtain an extract (pulverization-extraction method).

A superclinical extraction method is illustrated specifically below.
After Hematococcus alga has been dried and pulverized, the pulverized Hematococcus alga is filled in an extraction layer, an extraction is conducted with carbon dioxide in superclinical state as an extracting agent, and then carbon dioxide is removed to obtain an extract. Despite a substance in superclinical state has similar extraction ability as liquid, it has diffusion ability near that of gas and hence it has high extraction efficiency so that an extracting agent which is extraction solvent can be easily and completely removed without being remained. The superclinical extraction may be conducted by the method described in JP 2004-41147 A.

As the condition for superclinical extraction in case where carbon dioxide is used as an extracting agent, temperature is usually 31- 80 °C, preferably 31- 50 °C, pressure being 7.38 - 40 Mpa, preferably 10 - 38 Mpa. Incidentally, carbon dioxide of superclinical fluid is not restricted to fluid in superclinical state, that is, one in state where temperature and pressure exceeded the superclinical temperature and the superclinical pressure. It includes fluid whose temperature and pressure lie near the respective clinical points, the so-called fluid in sub-superclinical state.

In order to enhance extraction ability, an auxiliary solvent is added to superclinical fluid of carbon dioxide thereby the extraction ability can be enhanced. Examples of an auxiliary solvent include glycerin, ethanol, water and the like, and glycerin is preferred. The amount of an auxiliary solvent increases and decreases depending on the change in extraction velocity. For example, when the extraction time becomes long, the extraction velocity is lowered, and therefore it is recommended that auxiliary solvent is added to the superclinical fluid in great amount.

As Hematococcus which can be used in extraction, there can be used one pulverized and dried according to the conventional method. Pulverization and drying may be conducted according to the method described in WO 2002/077105 pamphlet whereby powdered one can be obtained. The powdered one may be filled as it is. Alternatively, it may be in advance formed into pellet, spherical or other shaped form and the shaped form may be filled. To powdered one and/or the shaped one are in advance added the above-described auxiliary solvent or an extraction-accelerating agent thereby the extraction ability may be enhanced.

An extraction-accelerating agent indicates oils and fats which are contained in algae such as Hematococcus alga or the like. By adding a similar substance in advance, the formation of porosity in the shaped form is promoted and its contact area with superclinical fluid becomes large thereby extraction ability is considered increased. As oils and fats, there are taken one or more of ones selected from triglyceride of middle chain fatty acid having 8 to 12 carbon atoms, rapeseed oil, corn oil, olive oil and the like, and triglyceride of middle chain fatty acid having 8 to 12 carbon atoms is preferred.

The pulverization-extraction method is specifically illustrated below.
The pulverization-extraction method is one wherein Hematococcus alga is pulverized, the inner substances are extracted with an organic solvent and the organic solvent is removed to obtain an extract. Pulverization of Hematococcus alga is conducted in water or an organic solvent. In case where Hematococcus alga has been pulverized in water, spray drying or the like is applied for drying. After removal of water, the residue is subjected to extraction using an organic solvent. Especially, the following method is suitable: After Hematococcus alga has been suspended in an organic solvent, the suspension is passed through a pulverizing machine to effect extraction together with pulverization. Solid matters are removed and then organic solvent is removed to obtain an extract. This method has characteristic features that since superfluous heating is not applied to Hematococcus alga and Hematococcus alga is not exposed to air during the extraction step, the oxidation of the extracted inner substances such as lipid, astaxanthin and the like is prevented and the amount of impurities formed during the production is very small. For example, the present applicant's earlier filed (Japanese Patent Application No. 2004-253525) method is taken.

As Hematococcus alga for use herein, wet or dry one may be used. A satisfactant and an antioxidant may be added depending on the necessity. Examples of a satisfactant include polyglycerin fatty acid ester, glycerin fatty acid ester, sucrose fatty acid ester, sorbitan fatty acid ester and propylene glycol fatty acid ester. Examples of an antioxidant include vitamin E (tocopherol), derivatives of vitamin E, tocotrienol, rosemary extract, vitamin C, derivatives of vitamin C, glutathion, phytic acid, catechinic acid, flavonoids, β-carotene, and the like.

Examples of an organic solvent which can be used for extraction are alcohols such as ethanol, methanol and the like, acetone, ether, hexane, benzene, chloroform, methylene chloride, preferably ethanol, methanol, acetone and hexane, more preferably acetone, ethanol and hexane.

As method of pulverizing a natural origin containing carotenoids in the present invention, the natural origin containing carotenoids is suspended in an organic solvent and the suspension may be subjected to wet pulverization using a pulverizing machine which permits the use of an organic solvent, for example, a high-pressure homogenizer, an ultrasonic grinder, a bead mill and the like. Preferably it is subjected to wet pulverization with a bead mill.

As the pulverization condition for bead mill, it is recommended that wet pulverization be conducted in a short time in order to suppress the deterioration of the extract. A retention time is 1- 30 minutes and a peripheral speed is 2 - 30 m/sec. A diameter of bead to be used is 0.2 - 5 mm and a temperature at which pulverization is conducted -10 - 50 °C.

As a method of removing solid matters, it may be conducted by the conventional manner, for example filtration under pressure, filtration under reduced pressure, spontaneous filtration or otherwise.

As a method of removing a solvent, it may be conducted according to the conventional manner. An organic solvent is removed from the filtrate using a vacuum-concentrating machine. In order to suppress the deterioration of the extract, it is recommended that application of superfluous heating be avoided.
It is conducted at 0 - 200 °C preferably 20 - 80 °C and it is better that one batch of treating time is a short. The solvent is removed in such amount that it can be treated for 0.5 - 20 hours, preferably 0.5 - 10 hours. For removing solvent as large as possible, the concentration of the solvent may be further reduced by a molecular distillation machine, thin membrane type centrifugal evaporator or the like.

As the form of astaxanthin to be used, there may be used the Hematococcus algae extract containing astaxanthin which was obtained by the above-described method, powders or an aqueous solution containing it, a dried product and pulverized product of *Phaffia* red yeast, Haematococcus green alga or oceanic bacteria. Among of them, the extract of Hematococcus algae is preferable from the viewpoints of easiness of handling and high astaxanthin content.

Astaxanthin is 3,3'-dihydroxy-β, β-carotene-4,4'-dione and has stereoisomers. Specifically, such three stereoisomers are known as (3R, 3'R)-astaxanthin, (3R, 3'S)-astaxanthin and (3S, 3'S)-astaxanthin. Any of them can be used in the present invention.

It is known that astaxanthin has not been observed having any mutagenicity and is highly safe compound and it has been widely used as food additive (Takahashi et al; toxicity test of Hematococcus alga. astaxanthin- Ames test, rat single dose toxicity test, rat 90-days repeat dose oral toxicity test- Journal of Clinical Therapeutic and Medicine, 20:86)

Unless otherwise described herein, astaxanthin includes astaxanthin and/or its ester. Furthermore, ester of astaxanthin includes monoester and/or diester.

As astaxanthin in the composition for body fat reduction in the present invention, there can be used at least one of free form, monoester form and diester form of astaxanthin.
The diester form is chemically and physically more stable than the free or monoester form and hard to be subjected to oxidative decomposition, because its two hydroxy groups are protected by ester bondage. However, when it is taken into the living body, it is considered quickly hydrolyzed into free astaxanthin by bioenzymes to exert its effect.

As a monoester of astaxanthin, there can be taken monoesters esterified with lower or higher saturated fatty acid, or lower or higher unsaturated fatty acid. Specific examples of lower or higher saturated fatty acid, or lower or higher unsaturated fatty acid include acetic acid, lauric acid, myristic acid, pentadecanoic acid, palmitic acid, palmitoleic acid, heptadecanoic acid, elaidic acid, ricinoleic acid, petroselinic acid, vaccenic acid, eleostearic acid, punicinic acid, licanoic acid, palynalic acid, gadolic acid, 5-eicosenoic acid, 5-docosenoic acid, cetolic acid, ercinoic acid, 5,13-docosadienoic acid, selacholic acid, decenoic acid, stering acid, dodecenoic acid, oleic acid, stearic acid, eicosapentaenoic acid, docosahexaenoic acid, linoleic acid, linolenic acid, arachidonic acid, etc
As a diester of astaxanthin, there can be taken diesters esterified with the same or different fatty acids selected from the above fatty acids.

Furthermore, as monoester of astaxanthin, there can be taken monoesters esterified with an amino acid such as glycine, alanine or the like; with a mono- or poly-carboxylic acid such as acetic acid, citric acid or the like; with an inorganic acid such as phosphoric acid, sulfuric acid or the like; with a saccharide such as glucoside or the like; with a glyco-fatty acid such as glycoglycero-fatty acid or sphingoglyco-fatty acid; with a fatty acid such as glycero-fatty acid, with glycero-phosphoric acid, etc. In case where it is to be considered, salt of the above monoester is included.

As a diester of astaxanthin, there can be taken diesters esterified with the same or different acids selected from the above lower saturated fatty acid, higher saturated fatty acid, lower unsaturated fatty acid, higher unsaturated fatty acid, an amino acid, a mono- or poly-carboxylic acid, an inorganic acid, a sugar, a glyco-fatty acid, fatty acid and glycero-phosphoric acid. In case where it is to be considered, salt of the above diester is included. As a diester of glycero-phosphoric acid, there may be taken saturated fatty acid ester of glycero-phosphoric acid or esters of glycero-phosphoric acid containing fatty acid selected from higher unsaturated fatty acid, unsaturated fatty acid and saturated fatty acid.

In order to enhance effect for astaxanthin and Hematococcus alga extract to reduce body fat in the present invention, one or more of an active ingredient, an adsorbent and a solubilizer can be incorporated into astaxanthin or Hematococcus alga extract.
Examples of an active ingredient include vitamin A substances, carotenoids, vitamin B substances, Vitamin C substances, vitamin D substances, Vitamin E substances, tocotrienol, glutathion, their derivatives and their salts: a-ribo acid, deoxyribonucleic acid, ribonucleic acid, adenosine triphosphate, adenosine monophosphate, glycyrrhizin, glycyrrhizic acid, guanine, xanthine, α- or γ-linolenic acid, eicosapentaenoic acid, succinic acid, estradiol, their derivatives and their salts: aspartic acid, α-hydroxy acid such as glycolic acid, lactic acid, malic acid, citric acid, salicylic acid and the like; an extract of deproteinized serum, spleen extract, placenta extract, crest extract, royal jerry, yeast extract, extract of lactic acid bacteria, extract of bifid bacteria, *Fomes Japonioucus* extract, carrot extract, *Swertia* extract, rosemary extract, phellodendron bark extract, garlic extract, Hinokitiol, cepharanthine, aloe extract, *Salvia splendens* extract, arunica extract, camonile extract, white birch extract, *hypericum* extract, *Eucalyptus* extract, *Xuan Fu Hua* extract, *patholobus suberectus Dunn* extract, Sanpenzu extract, moricortex extract, *Angerica* extract, *Bistorta* extract, *Sophora* extract, *Crataegus* extract, white lily extract, hop extract, wild rose extract, *Coix lacryma-jobi* extract; D-fraction, glycogen, Octacosanol, allicin, coenzyme Q₁₀, catechin; cystine, its derivative and salt; peptide; lysine, allyl sulfide, biotin, pantothenic acid, collagen, elastin, keratin, their derivatives and salts thereof; hyaluronic acid, chondroitin sulfuric acid, dermatan sulfate, heparitin sulfate, heparin, keratan sulfate; lactic acid bacteria; minerals such as iron, morybdenum, calcium, zinc, selenium, manganese, cupper, iodine and the like; etc. Tocopherol, tocotrienol, a-ribo acid, coenzyme Q₁₀ and their derivatives are preferred.

As an adsorbent which can be incorporated into the composition for body fat reduction in the present invention, there may be taken saccharides such as lactose, sucrose and the like; sugar alcohols such as sorbitol, mannitol, xylitol, erythritol, maltitol and the like; inorganic compounds such as magnesium aluminometasilicate, hydrotalcite, Magaldrate, calcium phosphoric acid anhydride, calcium carbonate, salicic acid anhydride, silicon dioxide, talc and the like; celluloses, starches, gelatin and agar.
The composition of the present invention may be prepared by subjecting astaxanthin or Hematococcus alga extract, and/or an active agent and a solubilizer to wet or dry granulation. As wet granule, it may be prepared by the conventional method, for example spray drying, fluidized bed granulation, mixing granulation or freeze-drying.

As a solubilizer which can be incorporated into the composition for body fat reduction in the present invention, there may be taken a triglyceride of middle chain fatty acid, an eatable fat and oil, a fatty acid ester, glycerin, ethylene glycol and the like.
For preparing the composition of the present invention, astaxanthin or Hematococcus alga extract, and/or an active agent, and a solubilizer may be mixed together according to the conventional method. The mixture is absorbed on the above adsorbent and is subjected to granulation thereby the composition of the present invention may be also prepared. The presence of these adsorbent and solubilizer are effective to prevent the oxidation of astaxanthin because the contact area of astaxanthin with an air becomes small. They are also effective to protect astaxanthin until it reaches digestive part such as intestine and the like.

In the composition of the present invention, astaxanthin is contained in an amount of 0.001- 99.9 %, preferably 0.01 - 20 %, more preferably 0.1 - 10 % based on the total weight of the composition. In the composition of the present invention, Hematococcus alga extract containing astaxanthin is contained in an amount of 0.001 - 99.9 %, preferably 0.1-80 %, more preferably 1 - 50 % based on the total weight of the composition.

There are contained 0.05 - 2 parts by weight, preferably 0.1 - 1 part by weight of an active ingredient, 1 - 1000 parts by weight, preferably 2 -500 parts by weight of an adsorbent and 0.05 - 2 parts by weight, preferably 0.1 - 1 part by weight of solubilizer per 1 part by weight of astaxanthin. There are contained 0.01 - 1 part by weight, preferably 0.02 - 0.5 part by weight of an active ingredient, 1- 1000 parts by weight, preferably 2 -500 parts by weight of adsorbent and 0.05-2 parts by weight, preferably 0.1 - 1 part by weight of solubilizer per 1 part by weightof Hematococcus alga extract containing astaxanthin.

The amount administered or taken of the present composition is 0.2 - 100 mg, preferably 0.5 - 20 mg in terms of free astaxanthin for an adult per day. Oral administration or parenteral administration is conducted. The amount administered may vary in age, body weight and grade of symptoms of a patient to be administered and dosing form.

The terms "effect of reducing body fat" is the effect of decreasing body fat and the rate of body fat as well as the effect of preventing body fat and the rate of body fat from increasing despite no change in body weight. Practicing a light exercise periodically while the composition for body fat reduction in the present invention is administered or taken can further enhance the effect of reducing body fat. By administering or taking the composition for body fat reduction in the present invention, the increase in body fat can be prevented and consequently effect of preventing corpulence is exerted.

The reduction of an excessive body fat causes effect to treat, improve and prevent a disease the cause of which is said to be corpulence or excess of body weight, for example diabetes, arteriosclerosis, hyperpiesia, cancer, hyperlipemia, ryeumatism, hyperrucicemia, arthritis deformans, gout, cerebral accident, ischemic heart disease, respiratory injury, pancreatitis, cataract, Alzheimer's disease, allergic disease, aging, hidrosis, ischemic disease, complications of diabetes being kidney disease, nerve injury and retinopathy.
In nerve injury, it is effective in treating improving and preventing sudden bradyacusia , abnormality of eye and face (paralysis and pain), orthostatic hypotension, abnormality of perspiration, flux and obstipation (digestive trouble), dysuria, pain of membrum, pareatrophy, paresthesia, atrophy of muscle and anthrax. In eye injury, it is effective in treating improving and preventing cataract, simple retinosis, preproliferative retinopathy, proliferative retinopathy. Also, in ischemic disease, it is effective in treating, improving and preventing brain infarction and cardiac infarction.

As exercise for enhancing effect of reducing body fat in the present invention, aerobic exercise such as walking, bicycling, climbing, aerobics, machine training, aerobics in water, walking in water and so on may be taken. It may be taken 2 - 4 times for a week at the quantity of exercise of 30 minutes - 2 hours per one time.

The composition of the present invention may be formed into a medicament in such forms as tablet, sublingual tablet, pill, suppository, triturate, powder, fine granule, granule, capsule, microcapsule, injection, emulsion, patch and so on. For example, tablet may be prepared by uniformly mixing the composition of the present invention with a pharmaceutically acceptable carrier and tableting the mixture. Also, triturate, powder and granule may be prepared by subjecting the composition of the present invention and a carrier to dry granulation or wet granulation. Wet granule may be prepared by drying according to the conventional manner, for example spray drying, fluidized bed granulation mixing granulation or freeze-drying. Also, a chewable tablet or an intraoral disintegratable tablet may be prepared by the conventional manner depending on the necessity.

Triturate, powder, fine granule, granule and tablet may be prepared using an excipient such as lactose, glucose, sucrose mannitol, magnesium aluminometasilicate, synthetic hydrotalcite, anhydrous calcium hydrogen phosphate or the like; a disintegrant such as starch, sodium alginate, or the like; a lubricant such as magnesium stearate, talc or the like; a binding agent such as polyvinyl alcohol, hydroxypropylcellulose, gelatin or the like; a satisfactant such as fatty acid ester or the like; plasticizer such as glycerin or the like.

The composition comprising staxanthin or its ester as an effective ingredient in the present invention may be appropriately combined with sugar alcohols such as sorbitol, mannitol, xylitol, erythritol, maltitol and the like; saccharides such as lactose sucrose, maltitol and the like; inorganic excipients such as magnesium aluminometasilicate, hydrotalcite, magaldrate, anhydrous calcium phosphate, calcium carbonate and the like; binding agents such as starch, gelatin, methyl cellulose, polyvinylpyrrolidone, and the like; disintegrants such as starch, agar, crospovidone, crystalline cellulose and the like; lubricants such as silicon dioxide, talc, magnesium stearate, polyethylene glycoland and the like, flavors and sweetening agents thereby various forms of medicaments can be prepared. For example, the medicament is administered in such dosing forms as solid preparations such as tablet, intraoral disinteegratale tablet, capsule, granule, fine granule and the like and in such dosing forms as liquid preparations such as elixir, syrup and suspension.

In order to suppress decomposition and oxidation of astaxanthin or its ester, there can be added a substance having anti-oxidative ability as stabilizer can be added to the above compositions, if necessary. For example, one or two or more mixtures selected from such existing antioxidants as, e.g. vitamin A, vitamin B, vitamin C and vitamin E or derivatives these vitamins, tocotrienol, cysteine, glutathione, glutathione peroxidase, citric acids, phosphoric acids, polyphenols, nucleic acids, herb medicines, marine algae, inorganic substances can be added to the above compositions. It is desirable to administer them in a finely powdered form in order to enhance absorbability of free astaxanthin or monoester form.

If necessary, other antioxidant may be added to the compositions of the present invention. Antioxidant is not particularly limited. Any one can be applied if it has antioxidative action. There can be selected one, two or more of antioxidants from the group consisting of vitamin A substances such as retinol, 3,4-dihydroxyretinol and the like; vitamin B; vitamin C substances such as D-ascorbic acid, L- ascorbic acid and the like; Vitamin E substances such tocopherol, tocotrienol, vitamin E acetate, vitamin E succinate; vitamin E phosphates; coenzyme, flavonoid, tannin, ellagic acid, polyphenols, radical inhibitor, hydroperoxide decomposing agent, metal chelating agent, active oxygene removing agent; carotenes containing α- carotene, β-carotene, γ- carotene, and δ- carotene; tocoquinone; pharmaceutically acceptable salts thereof and mixture thereof

An injection may be prepared according to the conventional manner and by compounding the effective ingredient with one or more of adjuvant agents selected from pH adjusting agent, buffering agent, solubilizer, suspension, isotonizing agent, stabilizer, antiseptic depending on necessity.

Examples of a suspension include polysorbate 80, methyl cellulose, hydroxyethyl cellulose, sodium carboxymethyl cellulose, polyoxyethylenesorbitan monolaurate, gum arabic, powdered tragacanth and the like. Examples of a solubilizer include polysorbate 80, polyoxyethylene hydrogenated caster oil, nicotinic acid amide, polyoxyethylenesorbitan monolaurate, macrogol, caster oil fatty acid ethyl ester and the like. Examples of a stabilizer include sodium sulfite, sodium metasulfite and the like. Examples of an antiseptic include methyl hydroxybenzoate, ethyl p-hydroxybenzoate, sorbic acid, phenol, cresol, chlorocresol and the like.

The composition of the present invention in the medicament may be contained in an amount of 0.01 - 99.9 % by weight, preferably 0.1 -90 % by weight. It may be determined adequately depending on dose per day.

The composition of the present invention can be used to prepare a food and drink having an effect of reducing body fat.

As the form of a food and drink to be incorporated, healthy foods such as functional food, food for specified health uses and supplemental food or general food and drink are taken.
As the forms of general foods and drinks, there may be taken an example of adding the composition of the present invention to general foods and drinks such as margarine, butter, butter sauce, cheese, raw cream, shortening, lard, ice cream, yogurt, diary products, meat sauce products, fish products, fried potato, potato chips, popcorn, a seasoned powder for spinkling over rice, chewing gum, chocolate, pudding, jelly, gumi-candy, candy, drops, caramel, sponge cake, cake, doughnut, biscuit, cookie, cracker, etc., macaroni, pasta, salad oils, instant soup, dressing, egg, mayonnaise, miso., etc., or carbonated or non-carbonated drinks such as fruit drinks, refreshing drinks, sports drinks, etc., non-alcoholic drinks such as tea, coffee, cocoa, etc., or liquors such as liqueur, medical liquor, etc.

When the food and drink of the present invention are used as healthy foods such as functional foods, food for specified health uses and supplemental foods and the like, their forms may be the same as the above-described medicament forms. There may also be used milk protein, soybean protein, egg albumin protein, etc., or their decomposed material such as albumen oligopeptide, soybean hydrolyzate, mixture of amino acid unit. The food can also be formed into natural liquid foods, semi-digested nutritional foods and nutritional foods, drinks, capsules or enteral nutrients, etc. combining with sugars, fats, trace elements, vitamins, emulsions, flavors, etc. For the drink form, such material can be combined with the drink as nutritional additives such as amino acids, vitamins, minerals, etc., and sweetening agents, spices, flavors, pigments, etc., in order to keep a balance in the nutrients or to impart good taste when taking.

In case where the composition of the present invention is incorporated into foods and drinks, it can be processed with raw materials of the general food and drink by usual methods. The quantity compounded of the composition of the present invention is not particularly restricted and it may be varied depending on the food form and so on. It may be generally 0.00001- 10 % by weight, preferably 0.00001 - 5% by weight and it is adjusted to necessary quantity to exert the effect of reducing body fat. The quantity for usage can be selected appropriately depending on the kind of food and drink by persons having ordinary skill in the art. Although the combining quantity of the composition of the present invention may vary depending on the food form and so on, generally it is desirable that the combining quantity as free astaxanthin lies in a range of 0.2 - 100 mg, preferably 0.5 - 20 mg per day for an adult.

In case where the food of the present invention is used as nutritional supplements or functional foods, the form may be the same as that of the above medicament. The form of the food in the present invention is not restricted to them. Also, in order to reduce the amount of body fat in animals, the composition of the present invention may be fed to them as feed.

The present invention is illustrated in more detail by the following examples, but it is needless to say that the present invention is not restricted thereto.

### [Preparation of Hematococcus alga extract]

80 Kg of dried and unpulverized Hematococcus alga powder (a product of Asta Real AB Company, astaxanthin content of 3.7 %) and 2 kg of mixed vitamins (a product of Riken Vitamin, E700) were dispersed in 120 kg of acetone, and the resultant dispersion was subjected twice to pulverization treatment a using anti-explosive type of bead mill tester [DYDO-MILL KDL-25BC, a product of WAB company]wherein bead mill having a diameter of 1 mm was compacted at a filling rate of 85 % at room temperature, cooling when required under disc peripheral speed of 10 m/s and a flow velocity of 100 kg/hr to extract a lipid fraction containing carotenoid at the same time.
The total amount of crushed suspension was subjected to sunction filtration to recover an extraction filtrate Furthermore, extraction residue was rinsed three times with 40 kg of acetone to extract lipid ingredients containing carotenoid completely. Total amount of the extraction filtrate obtained was concentrated under reduced pressure (100 torr, 45 °C) to remove acetone and to obtain Hematococcus alga extract containing 5.0 % of astaxanthin in terms of the free form. Hematococcus alga used here was one cultivated using a hermetic type of cultivation apparatus.
This Hematococcus alga extract was formed into a capsule containing 6 mg of astaxanthin in terms of the free form (test capsule) and used for test. As placebo capsule (control capsule), a capsule containing 0 mg of astaxanthin and having the same shape was used for test.

### [Person to be tested and intake method]

32 persons to be tested who were healthy women 23 - 57 years old were divided at random into two groups i.e. test and control groups, each consisting of 16 persons. The allocation of persons to be tested into test and control groups was conducted in a double-blind method not so as to know persons to be tested and persons participated in test the fact that each of persons to be tested belongs to which group.
Each of persons to be tested in test group took two test capsules a day after supper while each of persons in control group took two placebo capsules a day after supper. The intake period of term (test period of term) was 6 weeks. The rate of body fat and body weight were measured at the beginning (0 week) and the completion (6 weeks) of test for all persons. The rate of body fat was measured with a weighing meter [TBF-511, a product of Tanita Co. Ltd.]

### [Load of exercise in test period of term]

Exercise loaded on all persons to be tested in test period of term was walking. The quantity of exercise was determined by a maximum heart rate countering method.
Walking was taken continuously for 40 minutes at 60-70 % of maximum heart rate for persons having a maximum oxygen intake of more than 40 ml/kg body weight/min. and at 50 - 60 % of maximum heart rate for persons having a maximum oxygen intake of less than 40 ml/kg body weight/min. The walking was taken three times for a week.

**[Table 1] Change in an average body fat rate (%)**

| | Before test | After test | Change in an average body fat (%) |
|---|---|---|---|
| Test Group | 27.6±3.2 | 26.6±2.9 | -3.6 |
| Control Group | 26.6±3.1 | 26.8±3.6 | 0.8 |

| | | | |
|---|---|---|---|
| A: p<0.05 (t-test, control group vs test group) | | | |

**[Table 2] Change in an average body weight (Kg)**

| | Before test | After test | Change in an average body weight (%) |
|---|---|---|---|
| Test Group | 55.7±5.4 | 55.5±5.1 | -0.4 |
| Control Group | 54.1±6.3 | 54.4±6.3 | 0.6 |

| | | | |
|---|---|---|---|
| A: p<0.05 (t-test, control group vs test group) | | | |

Table 1 indicates the change in an average body fat rate and Table 2 does the change in an average body weight. No appreciable change in body weight was seen in the both groups.
The rate of body fat in the test group changed from 27.6 % to 26.6 %, indicating that body fat decreased by 3.6 %. Contrary thereto, the rate of body fat in the control group changed merely from 26.6 % to 26.8 %, indicating that no appreciable difference was seen.
It is apparent from this result that the rate of body fat decreases by intake of astaxanthin.

### [Preparation Example 1] Tablet

The following ingredients were uniformly mixed together in the composition ratio and powdered by dry method. Thereafter, the resultant powder was formed into tablets, each containing 300 mg of astaxanthin.

| | |
|---|---|
| Hematococcus alga extract | 300 mg |
| Lactose | 70 mg |
| Starch | 70 mg |
| Sodium caseinate | 6 mg |
| Gelatin | 6 mg |
| Cellulose | 109 mg |
| Silicon dioxide | 3 mg |
| Sucrose fatty acid ester | 6 mg |

Hematococcus alga extract contains 5 % by weight of astaxanthin in terms of free form.

### [Preparation Example 2] Soft capsule

Hematococcus alga extract (astaxanthin content of 5 % by weight) was mixed with an eatable oil and fat and filled in the outer shell of capsule consisting of the following ingredients according to the conventional method to make soft capsules, each 300 mg of weight.

| Inner filling | |
|---|---|
| Hematococcus alga extract | 20 mg |
| Eatable oil and fat | 150 mg |
| Outer shell | |
| Gelatin | 100mg |
| Glycerin | 30 mg |

### [Preparation Example 3] Drink

The following ingredients were compounded together and 10 kg of water was added according to the conventional method to prepare a drink.

| | |
|---|---|
| Aqueous solution of Hematococcus alga extract* | 25 g |
| Liquid sugar | 4000 g |
| Citric acid | 1 g |
| Vitamin C | 50 g |
| Vitamin E | 50 g |
| Cyclodextrin | 150 g |
| Potassium chloride | 25 g |
| Magnesium sulfate | 5 g |

| | |
|---|---|
| *Aqueous solution of Hematococcus alga extract (astaxanthin content of 1 %) prepared by the method of Example described in JP 2001-316601 A | |

### [Preparation Example 4] Stick gnanule

The following ingredients were compounded and granulated according to the conventional method to prepare stick granules, each containing 5 g.

| | |
|---|---|
| Astareal powder | 5% |
| Vitamin B mix | 1% |
| Nicotinic acid | 0.1 % |
| Panthothenic acid | 0.1 % |
| Taurine | 10% |
| Glutamic acid | 1 % |
| GABA | 0.01 % |
| Aspartic acid | 0.5 % |
| BCAA | 10 % |
| Citric acid | 10 % |
| Vitamin C | 10 % |
| γ-Oryzanol powder | 0.15 % |
| CMCNa | Balance |
| Dextrin | Balance |

| | |
|---|---|
| *Powder of Hematococcus alga extract (astaxanthin content of 1 % by weight) | |

## Claims

1. A composition for body fat reduction which contains astaxanthin as an effective ingredient.

2. A composition for body fat reduction which is obtained by pulverizing *Haetmatococcus* alga and subjecting pulverized *Haetmatococcus* alga to solvent extraction.

3. A composition for body fat reduction which comprises incorporating one or more of an active ingredient, an adsorbent and a solubilizer into any one of the compositions claimed in Claims 1 and 2.

4. A body fat reducing agent containing any one of compositions claimed in Claims 1 to 3.

5. A food and drink for body fat reduction which contains any one of compositions claimed in Claims 1 to 3.

6. A process for reducing body fat which comprises administering or taking to a human any one of compositions claimed in Claims 1 to 3.
